# EUROPEAN PATENT SPECIFICATION

(11) **EP 3 085 391 B1**
(45) Date of publication and mention of the grant of the patent: **15.12.2021**
(21) Application number: 15164360.8
(22) Date of filing: 21.04.2015
(51) Int. Cl.: A61L 2/10

(54) **DISINFECTION DEVICE AND USE THEREOF**
DESINFEKTIONSVORRICHTUNG UND VERWENDUNG DAVON
DISPOSITIF DE DÉSINFECTION ET UTILISATION DE CELUI-CI

(43) Date of publication of application: 26.10.2016
(73) Proprietor: D Lite bvba, 3970 Leopoldsburg (BE)
(72) Inventor: Corthouts, Diederik, 3970 Leopoldsburg (BE); Cruysberghs, Yves, 2600 Berchem (BE); Cruysberghs, Miguel, 3940 Hechtel-Eksel (BE)
(74) Representative: Arnold & Siedsma

(56) References cited:
- WO-A2-2007/089312
- GB-A- 2 498 541
- US-A1- 2012 107 184
- US-B1- 6 710 357
- US-B1- 8 378 323

## Description

### Field of the invention

The present invention relates to a disinfection device comprising a disinfection chamber, at least one source of UV radiation for irradiating said disinfection chamber and support means arranged in said chamber for supporting at an upper surface an object with a surface to be disinfected, wherein the upper surface contacts a first area of the surface of the object.

The invention further relates to the use of the disinfection device.

### Background of the invention

Disinfection and sterilization of objects and air is an important element to prevent the spread of pathogenic bacteria and other microorganisms and concomitant diseases. It is known that such pathogenic bacteria are present in hospitals, in medical practices of f.i. dentists and other sanitary buildings full of weakened people susceptible for infections by such bacteria and other microorganisms.

Irradiating with UV-radiation is a suitable method of disinfection. The UV-radiation is most suitably germicidal UV-radiation, also known as UV-C radiation, which has a wavelength of in the range of 100 nm to 280 nm and more particularly in the range of 200- 270 nm, such as 250-260 nm, for instance 254 nm. Such wavelengths are absorbed by organic material and more particularly individual pyrimidine and thymine bases of DNA. Germicidal light or radiation has been used for many years to inactivate and kill organisms found in air and water and to sterilize surfaces. A photocatalyst is suitably added, since it enhances the disinfecting power of ultraviolet radiation. The photocatalyst is a nanoparticulate n-type semiconductor particles, such as titianium oxide (TiO₂) particles, but alternatively oxides may well be used.

GB2498541A1 discloses a disinfection device of the type mentioned in the opening paragraph. The support means is more particularly a UV-transparent supporting device, so that the object may be exposed to the UV-radiation from all sides. The support device is most suitably provided with the photocatalyst coating. This support means may be stationary or may comprise a conveyer belt arranged for moving the object. Rather than a single conveyer belt, a plurality of belts may be present and arranged in stairway configuration. An object that is transported on the uppermost conveyer belt will drop, at the end of the belt, on the conveyer belt immediately below, and is transported further, in the same direction (or possibly in the opposite direction). It will again drop on another belt, and its orientation on the belt may change. A side of the object may now rest on the belt, and the bottom of the object may become a side face. Such conveyer belts arranged in stairway configuration could alternatively be made of a matrial that is not UV-transparent.

The embodiments disclosed in GB2498541 have their disadvantages. The UV-transparent substrate is quartz glass. This material has the disadvantage that it is expensive and also very brittle. Furthermore, the presence of the photocatalyst coating entails the risk that the coating may be touched. Touching and more particularly cleaning may damage the coating. The use of a stairway configuration has the disadvantage that it renders the device bulky. Moreover, objects necessarily fall down from one belt to another belt in this embodiment. This may lead to damage to the object.

Similar prior art showing belts or rotation for moving the object to allow the first contact area to be exposed has been shown in US 6710357 B1 or WO 2007/089312 A2.

### Summary of the invention

It is therefore an object of the invention to provide an improved disinfection device of the type mentioned in the opening paragraph without the above mentioned disadvantages of the prior art.

In accordance with the invention, this object is achieved in a disinfection device as claimed in

Claim 1, comprising a disinfection chamber; at least one source of UV radiation for irradiating said disinfection chamber; support means arranged in said chamber for supporting at an upper surface an object with a surface to be disinfected, wherein the upper surface contacts a first area of the object's surface. Herein the device further comprises auxiliary support means comprising a plurality of support elements provided with an upper surface that is substantially dot- or line-shaped, wherein the support elements are dimensioned such that the object will always be supported by more than one support element. The auxiliary support means are movable, such the first area of the object's surface gets exposed by means of a movement of the auxiliary support means. The auxiliary support elements are selected from the group of pillars, plates or interconnected grid.

The movability of the auxiliary support means allows the irradiation of the surface of the object in two steps: first when lying on the support means, and secondly when held by the auxiliary support means. Such a construction turns out mechanically viable. Particularly, rather than creating large belts, the support elements of the auxiliary support means are herein defined to have an upper surface which is zero- or one-dimensional, particularly substantially as a dot or as a "line-shaped" element. The small dimension has been chosen to be small in comparison to the size of articles. Moreover, the distance between neighbouring support elements is also suitably small in comparison to the size of representative objects, such as tools used in chirurgy and/or dentistry.

The background is then that the object is moved by at least some of the support elements in a stable manner, without any transportation of the object. The object may be shifted upwards in the chamber, or alternatively may be translated slightly.

The movement according to the invention as defined in claim 1 is embodied as a movement in a direction normal to the upper surface of the support means, and more particularly in the vertical direction. Such a choice has the advantage that the support means and the auxiliary support means may be arranged in a complementary pattern. In this manner, the support elements of the auxiliary support means are suitably distributed between the portions of the support means. This results in a proper, rather regular distribution of the support elements. In this manner, objects with widely differing sizes and shapes may be taken up from the support means and put down on it again without any stability issues, and without risk that extensions of the object such as legs, needles etc, would end up between the support means and the auxiliary support means.

A further advantage of the vertical movement of the support elements is the adequate implementation. Particularly, moving means may be arranged in a bottom section of the chamber and/or below the chamber. In one suitable implementation, the movement of the support elements is arranged by means of air cylinders. In an alternative suitable implementation, the movement is effected with an electrical or mechanical actuator. Springs may be present to drive the air cylinders automatically back to their rest position, which is particularly the bottom position.

In one implementation, the auxiliary support means are arranged to carry out the same movement. Suitably, a single moving means is present for such auxiliary support means. For instance, there is one engine that starts or stops carrying out the vertical movement on the basis of a single control. This improves robustness and simplifies the design.

In an alternative implementation, a first and a second auxiliary support means are each provided with moving means, so that their movements may be driven independently from each other. This embodiment has the advantage that the vertical movement may be carried out selectively, for instance on the basis of a sensor measurement. Thereto, in one further embodiment, the disinfection device is provided with (i) a camera arranged for registration of an image of the object; (ii) analysis means for analysis of the image so as to identify the first areas of the object at which the object gets supported by the support means, and to identify further surface areas at which the object can be supported by support elements of auxiliary support means, and (iii) control means for selectively driving auxiliary support means corresponding to the further surface areas. This embodiment of selective movement of some of the auxiliary support means tends to reduce a risk that the object to be disinfected, particularly one with comparatively long legs and protruding parts, would fall or shift due to inappropriate vertical shifting.

In again a further embodiment, the at least one source of UV radiation is located in a cavity below the support means, which support means are configured for transmission of the UV radiation towards an object on the support means and/or the auxiliary support means. In this manner, UV radiation is emitted towards the object, at the side that is not easily irradiated. In one implementation, the cavity is arranged such, that emitted UV radiation will pass through spaces between the support means and the auxiliary support means. Most suitably, use is made of a backlighting construction, as for instance applied in liquid crystal displays. This backlighting construction furthermore enables emitting UV radiation from a major portion of if not substantially the entire bottom area of the disinfection device.

In another implementation, the support means has a discontinuous top surface, for instance in the form of a grid or a series of plates or a plurality of pillars. The support means further have a body to which said discontinuous top surface is connected. The cavity for the at least one source of UV radiation is present in the body. In this manner, the first areas of the object's surface can be irradiated most directly, therewith minimizing the chance that some areas of the object's surface might not be irradiated and thus not disinfected.

Preferably, the at least one source of UV radiation is embodied as at least one light emitting diode (LED). Such LEDs have a small size, enabling their arrangement at various locations in the disinfecting device. In one further implementation, control means are present and configured for driving and suitably blinking the LEDs. More particularly, a control mechanism is implemented wherein a first LED and a second LED are switched on and off in accordance with different patterns, for instance different blinking patterns. This allows that the overall power level needed remains within limits. Moreover, in one implementation, a first set of LEDs is switched on in dependence on the position of the auxiliary support means. More particularly, the first set of LEDs may be switched on only if the auxiliary support means are in a top position wherein they carry the object.

It is observed for clarity, that the support means may be embodied in different forms. The support means can for instance be a substantially rigid structure, as a body that extends laterally, wherein grooves are present that are arranged such that the support elements of the auxiliary support means fit into the grooves. The support means may alternatively be embodied as a plurality of plates or pillars. The combination of support means and auxiliary support means may then form a series of parallel plates or an array of pillars.

In one further implementation, the support means may also be provided with moving means. Such moving means are however driven independently from the auxiliary support means. A first advantage of this implementation is that the maximum vertical movement for the auxiliary support means can be decreased: rather than that the auxiliary support means can be shifted between a position above and a position below the support means, both the auxiliary support means may carry out the same shift. The desired positions of the auxiliary support means above and below the support means can then be obtained by movements of the auxiliary support means, the support means or both of them.

In a further embodiment, the disinfection device is provided with means for transportation of the object into and out of the disinfection chamber. Such means of transportation have the benefit that the disinfection chamber can be held as clean as possible. In one implementation hereof, the support means may be configured for a movement from a first location to a second location or vice versa, wherein the first location is within the disinfection chamber and the second location is outside the disinfection chamber. The movement of the support means may herein be limited to an upper surface thereof, on which an object may be disposed. The second location may for instance be a separate chamber within the disinfection device. The second location is alternatively a location outside the disinfection device. The means of transportation are in a first embodiment provided with an electric motor. Alternatively, use can be made of a mechanical actuator that carries out a predefined movement, when another action occurs, for instance the opening or closing of a panel door. In again an alternative embodiment, the means of transportation are embodied as flexible holding means, constituting at least a portion of an enclosure of the disinfection chamber. A user may then using the flexible holding means, for instance in the form of a hand shoe for gripping the object and bringing it outside the disinfection chamber.

Furthermore, the disinfection device may be provided with spraying means for spraying or sprinkling of the object. Herewith, the disinfection device may also be used as a sterilisation device. The spraying means are suitably present in the disinfection chamber, although they could alternatively be present in a separate sterilisation chamber. Drying means are suitably present for drying the object after the spraying. The drying means are most suitably embodied in the form of a heating means. Use can be made of sources of infrared radiation. In one further implementation, the disinfection device is further provided with collecting means for the sprayed liquid. This is for instance a container. Alternatively, the collecting means are embodied as a plurality of channels at a bottom side. This implementation has the advantage of locating the channels between the moving means for the auxiliary support means and suitably adjacent to at least some of the sources of UV radiation.

In one suitable implementation, the disinfection chamber is enclosed by an inner wall that is made of a material transparent to UV-radiation. At least some of the UV-radiation sources are located behind said inner wall, such that the UV radiation will go through the inner wall for irradiating an object in the disinfection chamber. This is a good way of ensuring that users will not touch the sources of UV-radiation. More preferably, a photocatalyst is present, as discussed before. Such photocatalyst is suitably also provided outside the disinfection chamber. This location prevent inadvertently rubbing or chemically damaging the photocatalyst material when cleaning the inside of the disinfection chamber. When using LEDs as sources of UV radiation, the LED is suitably provided with a package that is transparent to UV radiation and more preferably comprises photocatalyst material. For instance, the photocatalyst material may be provided as a coating on a cover of the LED package, which is for instance a plate of quartz glass. If desired, a series of LEDs could be integrated into such dedicated LED package with the said cover with a coating of photocatalyst material. The LEDs may herein be the LED devices as commercially available that also have their own semiconductor package.

Furthermore, the disinfection chamber is suitably provided with reflectors. Suitably, the support means and the auxiliary support means are provided with reflecting surfaces. Although advantageous, it may not always be practical that the entire surface of these means is reflective. One form of a reflecting surface is for instance a white surface. In a further implementation, therefore, the support means and/or the auxiliary support means are embodied in a white-coloured material, such as a thermally stable engineering plastic, for instance a polyolefin that may contain glass fiber or another filler material so as to provide desired strength.

The disinfection device may further be provided with any conventional means that a skilled person will expect for the type of disinfection device, such as an enclosure for the disinfection chamber, a housing for the disinfection device in its entirety and means for powering the device. The enclosure is suitably an inner wall of a UV transparent material, wherein at least The device is suitably provided with more than one operation mode, and with any type of user interface.

### Brief introduction of the figures

These and other aspects of the invention will be further elucidated with reference to the figures, in which:
Figure 1 shows a first embodiment, wherein the support means are two interlocking grids, in a first state;
Figure 2 shows this embodiment in a second state;
Figure 3 shows a detailed view of this embodiment in the first state;
Figure 4 shows a detailed view of this embodiment in the second state;
Figure 5 shows one possible embodiment for the moving means to actuate the transition between the first and the second state;
Figure 6 shows a side-view of a second embodiment of the support means, wherein the upper surface of the support means is substantially one-dimensional;
Figure 7 shows a top view of this embodiment;
Figure 8 shows a side-view of support means embodied as rotating rods, not in accordance with the invention;
Figure 9 shows a top view corresponding to Figure 8;
Figure 10 shows a side-view of a third embodiment of the support means of the invention, in which the support means are embodied as a grid and pillars;
Figure 11 shows a top view of this embodiment.

### Detailed description of illustrated embodiments

The embodiments will now be described in more detail at the hand of the figures. Like numbers refer to like elements (though not necessarily identical elements).

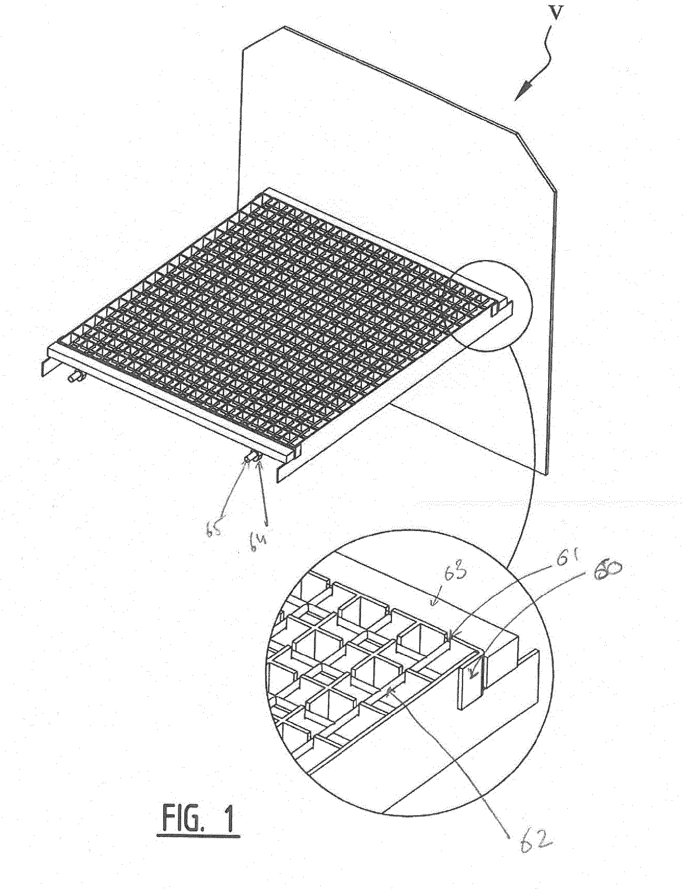

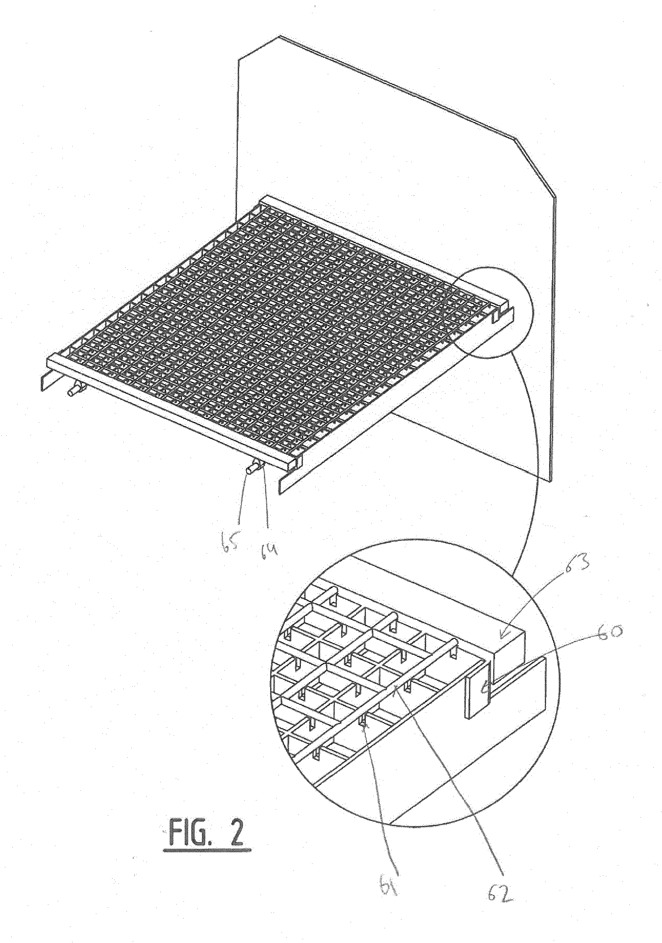

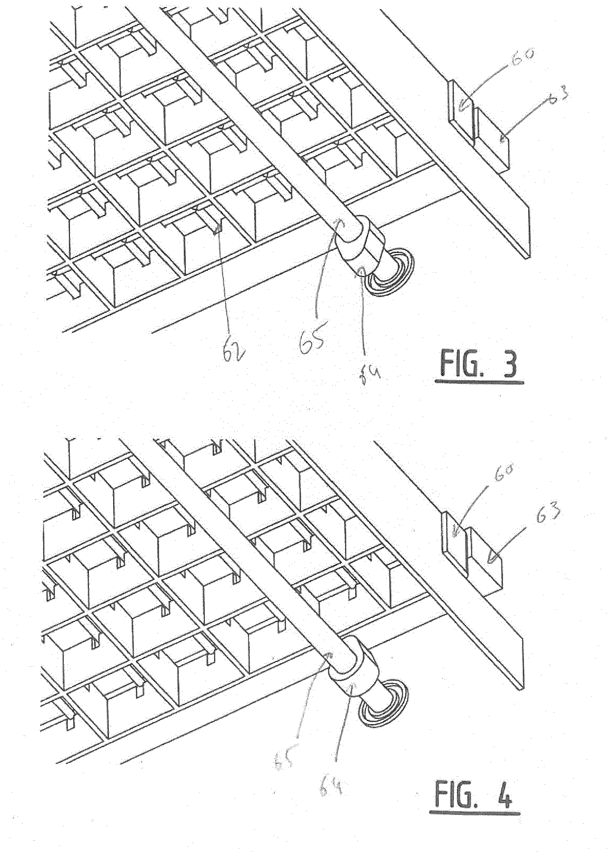

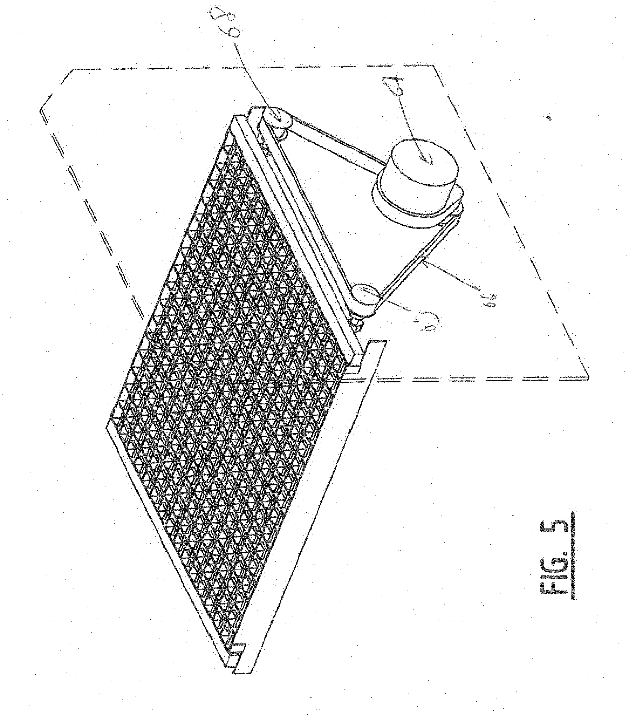

In an advantageous embodiment of the invention, which is depicted in Figures 1-5, the support means and auxiliary support means are embodied as two interlocking grids (60, 62). In a first state, such as is depicted in Figures 1 and 3, the upper surface of the first grid (60) is arranged above the upper surface of the second grid (62), and acts as support means for any articles positioned above the grids. In a second state, such as is depicted in Figures 2 and 4, the upper surface of the first grid (60) is arranged below the upper surface of the second grid (62), and the upper surface of the second grid acts as support means for any articles positioned above the grids. One of the advantages of this embodiment is its robust implementation and the option of driving to achieve a continuous up-and-down movement at higher frequencies, as will be discussed with reference to Fig. 5. Furthermore, the structure of the grid creates channels that can also be used for other purposes, such as the provision of the UV-C radiation from the bottom side and the generation of a ventilation flow in vertical direction (upwards or downwards) through the grid.

Several ways of designing two interlocking grids which can alternate between the two described states will be clear to the skilled person. In the depicted embodiment, the interlocking is achieved by the first grid (60) having grooves (61) at its upper side which fit elements of the second grid (62), where the second grid (62) is a simple wire grid, but alternatives (for instance wherein the second grid has grooves on its bottom side) are also possible.

In the first state, depicted in Figure 1 and Figure 3, the second grid (62) is resting in the grooves (61) of the first grid (60), and any articles in the chamber are resting on the upper side of the first grid. In the second state, depicted in Figure 2 and Figure 4, the second grid is lifted above the first grid, and any articles in the chamber are resting on its upper surface. As different areas of the articles are positioned above the upper surface of the first grid than are positioned above the second grid, by alternating between the two states all areas of the bottom surface of the articles may be irradiated.

One preferred mechanism to achieve the transition between the two states is depicted in more detail in Figures 3 and 4, wherein Figure 3 depicts the state in which the second grid is resting in the first grid (the first state) and Figure 4 depicts the state in which the second grid is lifted above the upper surface of the first grid (the second state).

The first grid is resting on a at least two rods (65). In the first state, these rods support the first grid (60) and also the second grid (62), both directly by supporting side element (63) and optionally also indirectly through its support of the first grid (which in the first state may support the second grid). The rods are equipped with at least one, and preferably at least two cam elements, which is (are) fixed onto the rod, arranged under the side element (63) of the second grid (62). If the rod is rotated, for instance over about 180°, the cam will be rotated along with it. It may then lift the side element (63) and thus the grid attached to this side element (i.e. the second grid). At this point, the surface of the rods themselves is no longer supporting the second grid, either directly or indirectly. The size and shape of the cam is chosen such that it will lift the second grid sufficiently to elevate the upper surface of the second grid (62) above the upper surface of the first grid (60), as can be seen in Figure 4. If the rod then continues to rotate, the cam will be rotated further along with it, and the second grid will again be lowered into the grooves (61) of the first grid.

The means for rotating the rods may be arranged within the chamber, but may more advantageously be arranged outside the chamber, as depicted in Figure 5. In this figure, the moving means are embodied by a belt mechanism: as a central actuating element (67) rotates, it engages a belt, which then engages elements (68, 69) attached to the ends of the rods (65), causing them (and the cams affixed to them) to rotate, thus occasioning the transitions between the first and second states. Preferably, the elements (68, 69) are similar in both diameter and other characteristics, such that the rods (65) are moved at a substantially identical speed.

This belt mechanism of rotating the rods as shown in Fig. 5 allows generating a continuous up-and-down movement of the second grid (62). Moreover, the duration at which an up-and-down movement cycle is completed, may be shortened. Frequencies of 1 or more cycles per second, preferably 10-100 cycles per second are feasible. Such higher frequencies enable that the object to the irradiated is not merely pushed up but will start to vibrate under the repeated impact (transmitted impulse) of the second grid (62). Such a vibration is understood beneficial for the UVC-disinfection in accordance with the invention. First of all, the vibration may remove any dust adhered to the surface, also in more delicate areas such as grooves and microcracks. Secondly, the vibration ensures that the created 'waste' from the disinfection does not adhere to the surface of the object, but brings it into the air. Such waste may be removed from the air by the provision of a flow of air. Thirdly, the object will be moved slightly under the vibration, therewith reducing the risk that some surface area remains that is not disinfected properly.

While in the depicted embodiment, the second grid is moved while the first grid remains stationary, alternatives are also possible, for instance embodiments in which both grids move (in opposite directions, either simultaneously, alternately, or variations thereupon) and embodiments in which the first grid moves and the second grid remains stationary.

In Figures 1-5, only one wall of the disinfection chamber is depicted to allow for a better view, but obviously the chamber is, in operation, preferably closed from all sides. Please note that while many elements - for instance the source or sources of UV radiation, and optionally reflecting means, spraying means and/or drying means - are not depicted in these figures, they may be and preferably are present. It will be clear to the skilled person where these elements may be advantageously positioned.

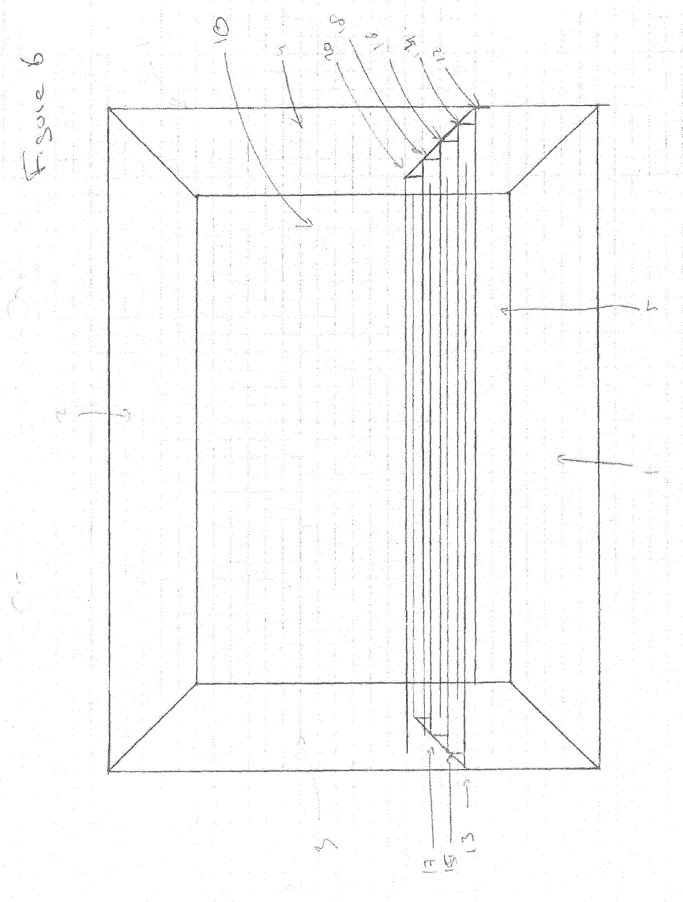

In Figures 6-11, the chamber (10) is depicted to have a box shape (i.e. to be a rectangular prism), but other shapes are also possible. In the depicted embodiments, the chamber has six walls: a bottom wall (1), a top wall (2), a left wall (3) a right wall (4), a back wall (5) and a front wall (6). However, not all walls are depicted in all figures.

Also present but not displayed are the source or sources of UV radiation, which may be located inside and/or outside the chamber. These may for example be present above and/or under the support means (12-20), preferably in a configuration that provides substantially even UV radiation to the area above the support means. Sources of UV radiation may also be present at the sides. Reflectors or reflecting surfaces may also be present to reflect the UV radiation and thus minimize the number of necessary sources and/or to optimize the distribution of UV radiation.

The skilled person will be able to choose an advantageous configuration of UV radiation sources to provide substantially even irradiation of the articles to be disinfected, in conjunction with the chosen support means. Other elements which may be present inside the chamber but which are not depicted in any of the figures are spraying means and/or drying means. It will be clear to the skilled person where these may be advantageously positioned.

In the embodiment of the invention depicted in Figure 6, the support means and auxiliary support means are embodied as elements with an upper surface that is essentially one-dimensional, i.e. line-elements. They may have an essentially one-dimensional side surface as well, but may also have a side surface with a more substantial side surface, depending for example on how sturdy the line elements must be to be able to support the articles to be disinfected.

In particular, in Figure 6, the line-elements are embodied as rods (or plates as according to the invention defined by the appended claims). Of these, about half (13, 15, 17, 19), which will be referred to as the left wall elements, are attached to the left wall (3) of the chamber (10) and end at a certain distance from the right wall (4) of the chamber, whereas the other half (12, 14, 16, 18, 20), which will be referred to as the right wall elements, are attached to the right wall (3) of the chamber and end at a certain distance from the left wall (3) of the chamber (10). Please note that the front wall (6) is not depicted in Figure 6 to allow an unfettered view of the inside of the chamber.

At least one of the sets of line-elements, and preferably both sets of line-elements, are attached to their respective walls in such a manner as to allow them to be displaced. This displacement may for example be substantially vertical, wherein for instance a rail may be used to allow each element to move up and down, preferably synchronously with the other line elements attached to the same wall. Other systems which allow for vertical translation of rod like (or, alternatively, sheet like; i.e. plates as according to the invention defined by the appended claims) elements will be obvious to the skilled person. In this way, no area of the articles is constantly in contact with the upper surface of a support element, and the entirety of the article's bottom surface may be irradiated.

To allow for even irradiation of the articles to be disinfected, the two sets of elements may alternately be translated. For instance, the right wall elements may be moved downward while the left wall elements are moved upward, after which the motion is reversed. Alternately, from a starting position in which both the left wall elements and the right wall elements are at an elevated position, the left wall elements may first move downward and then upward, and the right wall elements may subsequently go through a similar motion. This latter alternative may provide more stability for the articles supported by the support means. Yet another possibility is for just one of the sets of elements, for instance the left wall elements, to be movable, wherein this set is elements is translated between a state in which its upper surface is above the upper surface of the other set of elements, and a state in which its upper surface is below the upper surface of the other set of elements.

Any procedure may be chosen, as long as the result is that the articles are alternately supported by the left wall elements and the right wall elements. Optionally, there may also be moments in which the articles are supported by both sets of support elements simultaneously. In such a way, no area of the articles is constantly in contact with the upper surface of a support element, and all areas of the bottom surface of the articles may be disinfected with UV radiation.

As discussed with respect to Fig.5, also this embodiment allows increasing the frequency of movement, so as to arrive in a situation, in which the object to be disinfected is effectively vibrated under a regular impact of either one or both grids, so as to achieve a combination of cleaning and disinfection in optical and mechanical manner. However, it may be easier to provide such highfrequency implementation in the embodiment of Fig. 5.

Furthermore, the right and left wall elements may well be provided with means for applying charge. Particularly, the wall elements are charged oppositely, so as to constitute electrodes of opposite polarity. Alternatively, at least a portion of the wall elements is charged, with a counter electrode arranged above the grid. The use of electrical charge is known to be suitable for attracting dust and other particles and therewith clean air. The application hereof in combination with UV-C disinfection enables that particles and other matter that is removed from the surface of an object will not just be dispersed into the air, but attracted to the electrodes. In one suitable implementation, the thus created electrodes are merely charged temporarily, for instance to carry out air cleaning after the disinfection of the object's surface by means of UV-C radiation. Though the implementation of right and left wall elements appears highly suitable for their use as electrodes, it is not excluded that other embodiments could be used thereto as well.

The elements may also be able to be moved in a pivoting manner, for instance because they are attached to the respective walls with a hinge or a similar implement. In this case, in operation, first the left wall elements may be pivoted "out of the way", preferably toward the bottom of the chamber, then returned to the horizontal position in which it serves as support means for the articles to be disinfected, after which the right wall may be first pivoted "out of the way", preferably toward the bottom of the chamber, then returned to the horizontal position in which it serves as support means for the articles to be disinfected.

Yet another alternative may be for the support elements to be able to be retracted out of the chamber. The skilled person will be able to envision other alternatives yet, and will be able to determine which embodiment is most suitable.

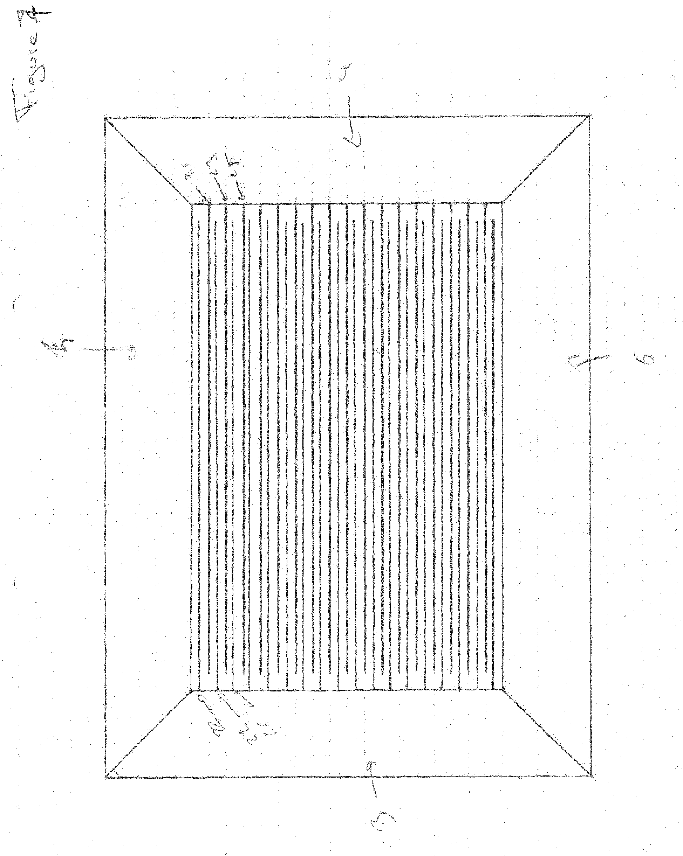

While Figure 6 displays only a limited number of elements, there may of course be fewer or more elements, for instance such as displayed in the top view seen in Figure 7. The number and spacing of the elements may be determined by the skilled person on the basis, for example, of the general weight, size and shape of the articles to be disinfected. For instance, for larger articles, fewer elements may be needed, but the elements may need to be sturdier to support the added weight; whereas for very small articles, the elements may need to be more closely spaced and hence more numerous, but they may not need to be as thick and/or sturdy.

In Figure 7, the front wall (6) is now visible, but the top wall (2) is not, in order to provide an unfettered view of the contents of the chamber (10). In this view, the fact that the elements have an essentially one-dimensional upper surface can be seen clearly, as well as the fact that they are alternately attached to the left or to the right walls of the chamber. While an alternating arrangement is not strictly necessary, it is the most convenient and stable arrangement. The skilled person will be able to judge which arrangement is suitable for the specific articles he or she wants to disinfect. While the elements are shown to extend across the longer side of the chamber, this need not be the case.

The moving means which make the movement of the line elements possible are not shown in the figures. They may be either inside or outside the chamber, or alternately included in the walls of the chamber. The skilled person will be able to envision many alternatives to achieve the desired motion in an efficient manner, as well as a suitable control system.

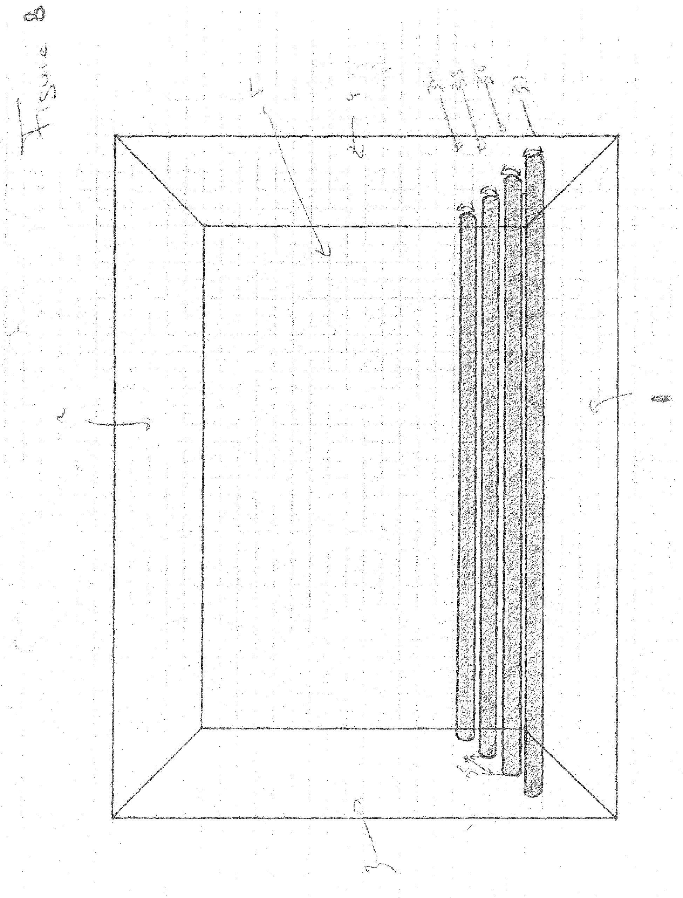

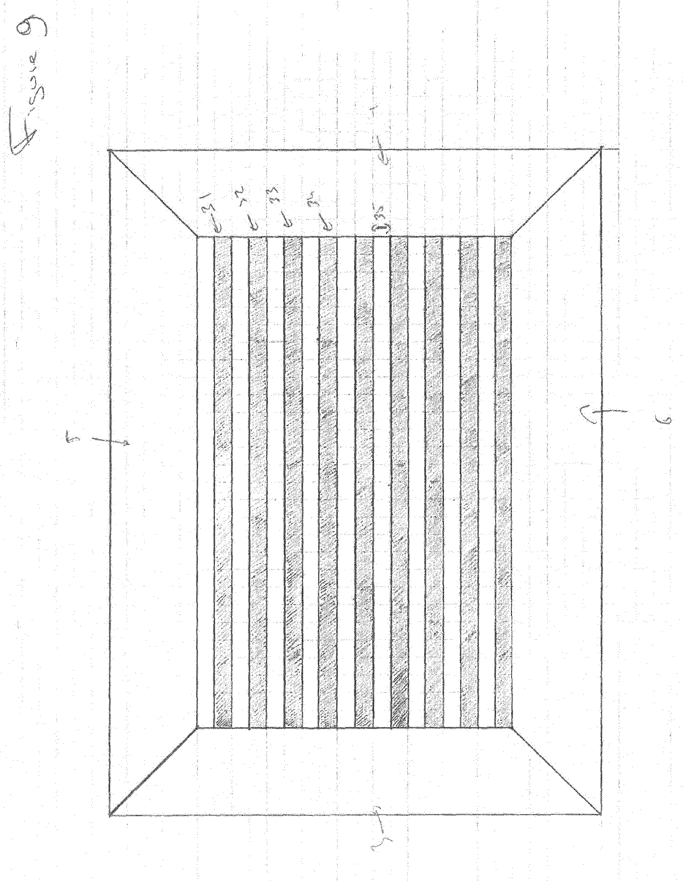

Figures 8 and 9 show an support means not in accordance with the claimed invention. In this instance, the support means are embodied as rods with a more substantial diameter, preferably with a substantially circular cross section. For stability, these rods are preferably attached to two opposing walls, for instance, as depicted, to both the left wall and the right wall of the chamber. These rods are provided - through moving means not depicted in these figured - with the ability to rotate, preferably around their central axis. The rotation need not be a full rotation - it may also only be possible for the rods to move a certain number of degrees smaller than 360 degrees, both clockwise and counter-clockwise.

As with Figures 1 and 2, Figure 8 does not depict the front wall, while Figure 9 does not depict the top wall. This is to allow an unfettered view of the contents of the chamber. The source or sources for UV radiation are also not depicted. It will however be clear to the skilled person, depending on the specifics of the embodiment, where the source or sources of UV radiation may advantageously be positioned.

There are several ways in which this particular embodiment may operate. A first manner, which would for instance be appropriate for substantially flat articles, would preferably employ rods (31-34) which have a diameter that is substantially smaller than the size of the articles to be irradiated and which are preferably separated by a certain distance (35) smaller than the smallest dimension of the articles, so as to prevent the articles from falling through. In this embodiment, all rods, or a certain subsection of rods supporting an article, are rotated in the same direction (either clockwise or counter-clockwise), which transports the articles resting on them along one direction in the chamber. The movement may be in one direction, but may also be of the back-and-forth type.

Using this method, a first area of the underside of the articles will be resting on the rods at a first time, leaving a second area exposed to radiation, while at a later time a different area of the underside of the articles, which may overlap with or be comprised within the second area, is resting on the rods, leaving the rest of the underside (preferably including the first area) exposed to radiation. In such a way, the entire underside of the articles can be exposed to UV radiation over time.

The system with the turning rods may also be employed in a different manner, which may be preferable for articles which are more boxy in shape. Preferably, in this variation of this embodiment, the distance between the rods may be only slightly smaller than the order of magnitude of the size of the objects. In this implementation, the articles are not resting on the rods but rather are supported between two adjacent rods. Turning the rods in pairwise opposite direction (i.e. for instance rods 31 and 33 clockwise and rods 32 and 34 counter-clockwise) may cause the articles to flip. For instance, an object positioned in a first orientation between rods 32 and 33 may, due the rotation of the rods, be transferred to a position between rods 31 and 32 (or 33 and 34) in a different orientation. By flipping the articles in such a manner a certain number of times, a substantially equalized distribution of radiation may be achieved.

Not all rods may need to rotate to be able to use this system. It may furthermore be advisable in this embodiment for the rods to have a somewhat roughened surface, which increases the friction between the rods and the articles.

Figure 9 depicts a similar embodiment from the top. To display the interior of the chamber, the top wall 2 is not depicted. More rods are depicted here than in Figure 8; it should be clear to the skilled person that different numbers and sizes of rods, as well as distances between rods, are appropriate for use with different articles to be disinfected; and the skilled person will be able to select a suitable configuration depending on the size and shape of the articles to be disinfected.

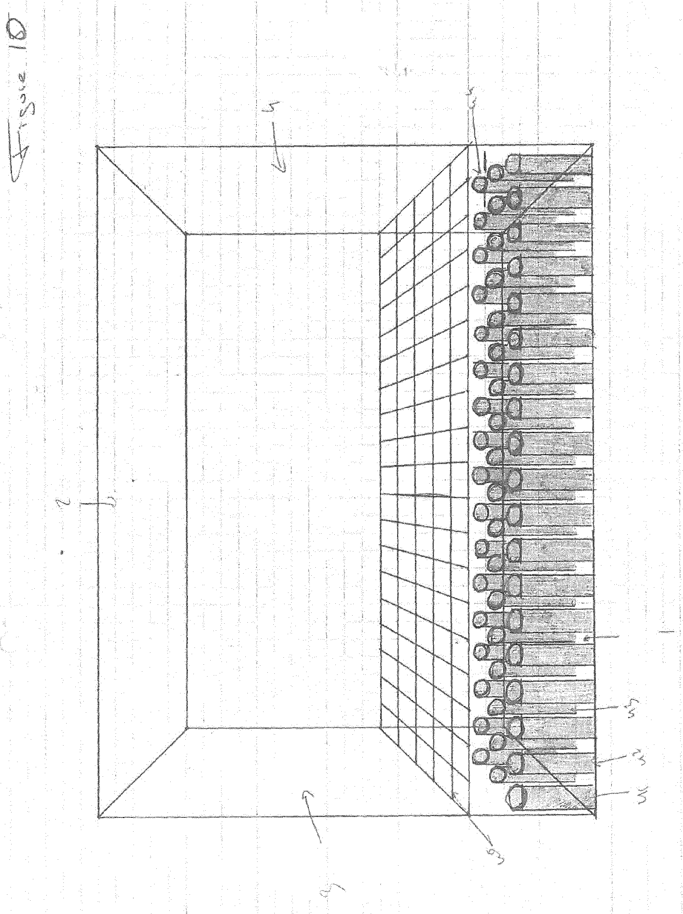

Figure 10 depicts a side-view of yet another embodiment in accordance with the invention. Here, the support means are embodied as a grid (40), and the support elements are embodied as pillars (41-44). These pillars are movable in the vertical direction by moving means not depicted in the figures. Also not depicted are the source or sources of UV irradiation. The skilled person will be able to determine a suitable configuration of these depending on the particulars of the situation.

The pillars are depicted in a state in which articles placed in the chamber will be supported by the grid, as the upper surfaces of the pillars are arranged below the upper surface of the grid. If the pillars are moved upwards, such that the upper surfaces of the pillars are arranged above the upper surface of the grid, the system will be in a different state in which the articles are supported not by the grid but by the upper surface of the pillars. By moving the pillars up and down and in such a way alternating between the two different states, the entire underside of the pillars can be irradiated and thus disinfected, as the area of the articles supported by the upper surface of the pillars in one state does not overlap with the area of the articles supported by the upper surface of the grid in the other state. Alternately, the pillars may be stationary and the grid may be moved up and down to achieve the transition between the two different states. The pillars and the grid may also both be most in opposite directions.

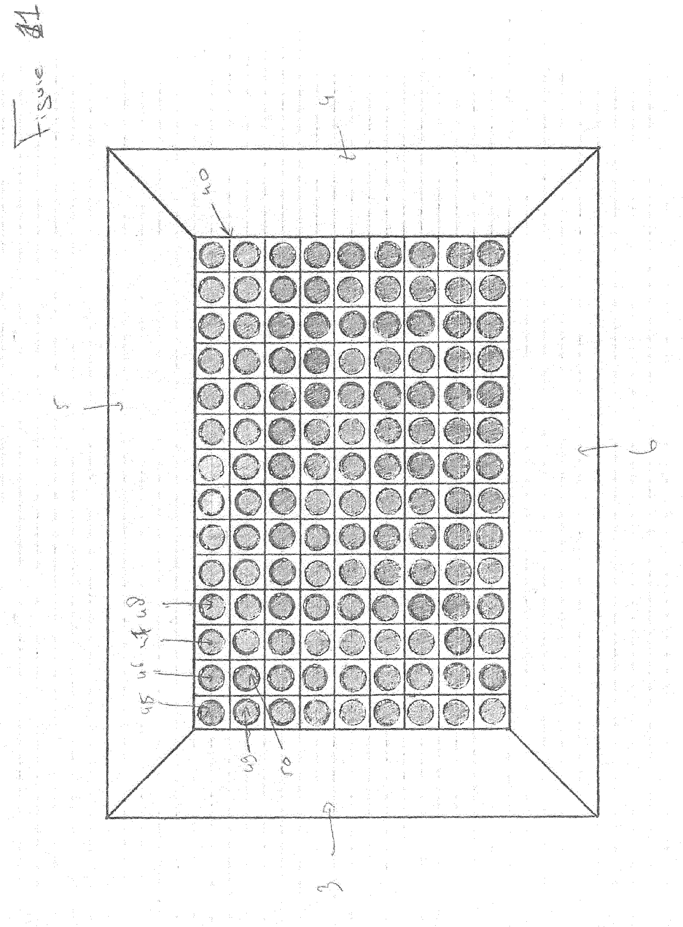

Alternatives to this embodiment are also possible without the presence of the grid. For instance, while in the embodiment described above the pillars are preferably moved in a synchronous manner, it may also be possible to divide the pillars into two sets. For instance, seeing the array of pillars as analogous to a checkerboard or chess board, the "white square" pillars could be seen as a first set and the "black square" pillars as a second set. To illustrate this, in Figure 11 (which shows a top view), pillars 45, 47 and 50 could be part of a first set, and pillar 46, 48 and 49 could be part of the second set. Other distributions into two sets are also possible to envision.

With such a configuration, the first set of pillars may for example be moved upward while the second set is moved downward and vice versa, and the articles are alternately supported by the upper surface of the first set of pillars and by the upper surface of the second set of pillars.

Alternately, it is sufficient for one of the two sets of pillars (for instance the first set) to be movable, while the second set stays still, if the first set moves between a first state in which its upper surface is arranged above the upper surface of the second set and a second state in which its upper surface is arranged below the upper surface of the second set, such that the articles are alternately supported by the upper surface of the pillars in the first set and the upper surface of the pillars in the second set. In this manner, the entire bottom surface of the articles may be disinfected over time, as no area of the articles is in constant contact with a support element.

In this alternative of the embodiment, the pillars are preferably spaced closer together than the smallest dimension of the articles to be disinfected, and/or the pillars would preferably have a diameter smaller than the smallest dimension of the articles to be disinfected, as it would be disadvantageous if the articles could fall between the pillars. The skilled person will be able to determine which size of pillars and which distance between them is suitable for the articles to be disinfected.

Figure 11 shows a top view of the embodiment described above, with the grid (40) and a plurality of pillars (45-50). The top wall is not depicted to allow an unfettered view of the contents of the chamber. The pillars are movable in the direction coming out of the page, by moving means not depicted in the figures.

It will be clear to the skilled person that the number, diameter, and distance between the pillars, as well as the dimensions and characteristics of the grid, may be modified to be suitable to the size and shape of the articles to be disinfected.

## Claims

1. Disinfection device configured for disinfection of a surface of an object, comprising:
- a disinfection chamber (10),
- at least one source of UV radiation for irradiating said disinfection chamber,
- support means (60; 12, 14, 16, 18, 20; 40) arranged in said chamber for supporting at an upper surface the said object, wherein the upper surface contacts a first area of the object's surface,
**characterized in that**
- the device further comprises auxiliary support means (62; 13, 15, 17, 19; 41-50) comprising a
plurality of support elements (62; 13, 15, 17, 19; 41-50) that are provided with an upper surface
that is substantially dot- or line-shaped, said support elements (62; 13, 15, 17, 19; 41-50) are
chosen from pillars (41-50), plates (13, 15, 17, 19) and/or constitute an interconnected grid (62), wherein the support elements (62; 13, 15, 17, 19; 41-50) are dimensioned such that the object will always be supported by more than one support element (62; 13, 15, 17, 19; 41-50) and
- the auxiliary support means (62; 13, 15, 17, 19; 41-50) are movable in a first direction
extending normal to the upper surface of the support means (60; 12, 14, 16, 18, 20; 40) , such the first area of the object's surface gets exposed by means of a movement of the auxiliary support means (62; 13, 15, 17, 19; 41-50). .

2. Disinfection device as claimed in claim 1, wherein the auxiliary support means (62; 13, 15, 17, 19; 41-50) are movable upwards and downwards, so that in a first state the upper surfaces of the support elements (62; 13, 15, 17, 19; 41-50) are arranged above the upper surface of the support means (60; 12, 14, 16, 18, 20; 40) , while in a second state the upper surfaces of the support elements (62; 13, 15, 17, 19; 41-50) are arranged below the upper surface of the support means (60; 12, 14, 16, 18, 20; 40).

3. Disinfection device as claimed in any of the claims 1-2, further comprising moving means configured for moving the auxiliary support means (62; 13, 15, 17, 19; 41-50) in a continuous up-and-down movement, particularly at a frequency sufficient to bring the object in a state of vibration.

4. Disinfection device as claimed in any of the claims 1-3, wherein the support elements of a first set are separate from each other and are embodied as pillars (41-50).

5. Disinfection device as claimed in claim 4, wherein the pillars are arranged in an array.

6. Disinfection device as claimed in any of the claims 1-3, wherein the support elements of a first set are separate from each other and are embodied as plates (13, 15, 17, 19).

7. Disinfection device as claimed in any of the claims 1-3, wherein the support elements are mutually interconnected to form a grid (62).

8. Disinfection device as claimed in any of the preceding claims 1-7, wherein at least one source of UV radiation is located in a cavity below the support means (60; 12, 14, 16, 18, 20; 40) which support means (60; 12, 14, 16, 18, 20, 40) are configured for transmission of the UV radiation towards an object on the support means (60; 12, 14, 16, 18, 20; 40) and/or the auxiliary support means (62; 13, 15, 17, 19; 41-50).

9. Disinfection device as claimed in claim 8, wherein the support means (60; 12, 14, 16, 18, 20; 40) have a discontinuous top surface and a body to which said discontinuous top surface is connected, and wherein the discontinuous top surface is in the form of a grid, a series of plates or a plurality of pillars.

10. Disinfection device as claimed in any of the preceding claims, further comprising:
- a camera arranged for registration of an image of the object,
- analysis means for analysis of the image so as to identify the first areas of the object at which the object gets supported by the support means (60; 12, 14, 16, 18, 20; 40) , and to identify further surface areas at which the object can be supported by support elements of auxiliary support means (62; 13, 15, 17, 19; 41-50), and
- control means for selectively driving auxiliary support means (62; 13, 15, 17, 19; 41-50). corresponding to the further surface areas.

11. Disinfection device as claimed in any of the preceding claims, further comprising a coating of a photocatalyst, such as a semiconductor material, such as TiO₂.

12. Disinfection device as claimed in claim 11, wherein the photocatalyst coating is provided on a first side of a support that is transparent for the UV-radiation, which support further comprises a second side, wherein the first side faces at least one source of UV radiation and wherein the second side constitutes an inner wall of the disinfection chamber.

13. Disinfection device as claimed in any of the preceding claims, wherein the disinfection chamber is provided with reflectors for reflection of the radiation.

14. Disinfection device as claimed in any of the preceding claims, further comprising spray nozzles for spraying a liquid onto the object and therewith removal of any infectitious matter.

15. Use of the disinfection device as claimed in any of the preceding claims for the disinfection of an object.

## Patentansprüche

1. Desinfektionsvorrichtung, die für die Desinfektion einer Oberfläche eines Objekts ausgebildet ist, aufweisend:
eine Desinfektionskammer (10),
mindestens eine UV-Strahlungsquelle zur Bestrahlung der Desinfektionskammer,
in der Kammer angeordnete Tragmittel (60; 12, 14, 16, 18, 20; 40), um das Objekt an einer oberen Fläche zu tragen, wobei die obere Fläche einen ersten Bereich der Oberfläche des Objekts kontaktiert,
**dadurch gekennzeichnet, dass**
die Vorrichtung ferner Hilfstragmittel (62; 13, 15, 17, 19; 41-50) aufweist, die eine Mehrzahl an Tragelementen (62; 13, 15, 17, 19; 41-50) umfassen, die mit einer oberen Oberfläche versehen sind, welche im Wesentlichen punkt- oder linienförmig ist, wobei die Tragelemente (62, 13, 15, 17, 19; 41-50) Säulen (41-50) oder Platten (13, 15, 17, 19) darstellen und/oder ein miteinander verbundenes Gitter (62) bilden, wobei die Tragelemente (62; 13, 15, 17, 19; 41-50) so dimensioniert sind, dass das Objekt stets von mehr als einem Tragelement (62; 13, 15, 17, 19; 41-50) getragen wird, und die Hilfstragmittel (62; 13, 15, 17, 19; 41-50) in einer ersten Richtung, die sich senkrecht zur oberen Fläche der Tragmittel (60; 12, 14, 16, 18, 20; 40) erstreckt, beweglich sind, so dass der erste Bereich der Oberfläche des Objekts durch eine Bewegung der Hilfstragmittel (62; 13, 15, 17, 19; 41-50) freigelegt wird.

2. Desinfektionsvorrichtung nach Anspruch 1, wobei die Hilfstragmittel (62, 13, 15, 17, 19, 41-50) nach oben und nach unten bewegbar sind, so dass in einem ersten Zustand die Oberseiten der Tragelemente (62; 13, 15, 17, 19; 41-50) oberhalb der Oberseiten der Tragmittel (60; 12, 14, 16, 18, 20; 40) angeordnet sind, während in einem zweiten Zustand die Oberseiten der Tragelemente (62; 13, 15, 17, 19; 41-50) unterhalb der Oberseite der Tragmittel (60; 12, 14, 16, 18, 20; 40) angeordnet sind.

3. Desinfektionsvorrichtung nach einem der Ansprüche 1 bis 2, die ferner eine Bewegungseinrichtung aufweist, die so ausgebildet ist, dass sie die Hilfstragmittel (62; 13, 15, 17, 19; 41-50) in einer kontinuierlichen Auf- und Abwärtsbewegung bewegt, insbesondere mit einer Frequenz, die ausreicht, um das Objekt in einen Vibrationszustand zu versetzen.

4. Desinfektionsvorrichtung nach einem der Ansprüche 1 bis 3, wobei die Tragelemente eines ersten Satzes voneinander getrennt sind und als Säulen (41-50) ausgebildet sind.

5. Desinfektionsvorrichtung nach Anspruch 4, wobei die Säulen in einer Matrix angeordnet sind.

6. Desinfektionsvorrichtung nach einem der Ansprüche 1 bis 3, wobei die Tragelemente eines ersten Satzes voneinander getrennt sind und als Platten (13, 15, 17, 19) ausgebildet sind.

7. Desinfektionsvorrichtung nach einem der Ansprüche 1 bis 3, wobei die Tragelemente miteinander verbunden sind, um ein Gitter (62) zu bilden.

8. Desinfektionsvorrichtung nach einem der vorherigen Ansprüche 1 bis 7, wobei sich mindestens eine UV-Strahlungsquelle in einem Hohlraum unter den Tragmitteln (60; 12, 14, 16, 18, 20; 40) befindet, wobei die Tragmittel (60; 12, 14, 16, 18, 20, 40) so ausgebildet sind, dass die auf ein auf den Tragmitteln (60; 12, 14, 16, 18, 20; 40) und/oder den Hilfstragmitteln (62; 13, 15, 17, 19; 41-50) befindliches Objekt gerichtete UV-Strahlung hindurchgelassen wird.

9. Desinfektionsvorrichtung nach Anspruch 8, wobei die Tragmittel (60; 12, 14, 16, 18, 20; 40) eine diskontinuierliche obere Fläche sowie einen Körper aufweisen, mit dem die diskontinuierliche obere Fläche verbunden ist, und wobei die diskontinuierliche obere Fläche die Form eines Gitters, einer Reihe von Platten oder einer Mehrzahl an Säulen aufweist.

10. Desinfektionsvorrichtung nach einem der vorherigen Ansprüche, ferner aufweisend:
eine Kamera, die zur Erfassung eines Bildes des Objekts angeordnet ist,
Analysemittel zur Analyse des Bildes, um die ersten Bereiche des Objekts, an denen das Objekt durch die Tragmittel (60; 12, 14, 16, 18, 20; 40) getragen wird, zu identifizieren, und um weitere Oberflächenbereiche zu identifizieren, auf denen das Objekt durch Tragelemente von Hilfstragmitteln (62; 13, 15, 17, 19; 41-50) getragen werden kann, und
Steuerungsmittel zum selektiven Ansteuern von Hilfstragmitteln (62; 13, 15, 17, 19; 41-50) entsprechend den weiteren Oberflächenbereichen.

11. Desinfektionsvorrichtung nach einem der vorherigen Ansprüche, die ferner eine Beschichtung aus einem Photokatalysator, beispielsweise aus einem Halbleitermaterial, wie TiO₂, aufweist.

12. Desinfektionsvorrichtung nach Anspruch 11, wobei die Photokatalysatorbeschichtung auf einer ersten Seite eines für die UV-Strahlung transparenten Trägers vorgesehen ist, wobei der Träger ferner eine zweite Seite aufweist, wobei die erste Seite mindestens einer UV-Strahlungsquelle, vorzugsweise für die Bereitstellung von keimtötender (UV-C) Strahlung, zugewandt ist und wobei die zweite Seite eine Innenwand der Desinfektionskammer bildet.

13. Desinfektionsvorrichtung nach einem der vorherigen Ansprüche, wobei die Desinfektionskammer mit Reflektoren zur Reflexion der Strahlung versehen ist.

14. Desinfektionsvorrichtung nach einem der vorherigen Ansprüche, ferner mit Sprühdüsen zum Aufsprühen einer Flüssigkeit auf den Gegenstand und damit zum Entfernen von infektiösen Stoffen.

15. Verwenden der Desinfektionsvorrichtung nach einem der vorherigen Ansprüche zur Desinfektion eines Gegenstandes.

## Revendications

1. Dispositif de désinfection configuré afin d'assurer la désinfection d'une surface d'un objet, comprenant :
un compartiment de désinfection (10),
au moins une source de rayonnement UV destinée à irradier ledit compartiment de désinfection,
des moyens de support (60 ; 12, 14, 16, 18, 20 ; 40) agencés dans ledit compartiment afin de supporter ledit objet au niveau d'une surface supérieure, dans lequel la surface supérieure entre en contact avec une première zone de la surface de l'objet,
**caractérisé en ce que**
le dispositif comprend, en outre, des moyens de support auxiliaires (62 ; 13, 15, 17, 19 ; 41 à 50) comprenant une pluralité d'éléments de support (62 ; 13, 15, 17, 19 ; 41 à 50) qui comportent une surface supérieure qui est sensiblement formée de ligne ou de point, lesdits éléments de support (62 ; 13, 15, 17, 19 ; 41 à 50) sont choisis à partir de colonnes (41 à 50), de plaques (13, 15, 17, 19) et/ou constituent une grille d'éléments reliés les uns aux autres (62), dans lequel les éléments de support (62 ; 13, 15, 17, 19 ; 41 à 50) sont dimensionnés de telle sorte que l'objet soit toujours supporté par un ou plusieurs éléments de support (62 ; 13, 15, 17, 19 ; 41 à 50), et
les moyens de support auxiliaires (62 ; 13, 15, 17, 19 ; 41 à 50) peuvent être déplacés dans une première direction s'étendant normalement à la surface supérieure des moyens de support (60 ; 12, 14, 16, 18, 20 ; 40), de telle sorte que la première zone de la surface de l'objet est exposée suite à un mouvement des moyens de support auxiliaires (62 ; 13, 15, 17, 19 ; 41 à 50).

2. Dispositif de désinfection selon la revendication 1, dans lequel les moyens de support auxiliaires (62 ; 13, 15, 17, 19 ; 41 à 50) peuvent être déplacés vers le haut et vers le bas, de telle sorte que, dans un premier état, les surfaces supérieures des éléments de support (62 ; 13, 15, 17, 19 ; 41 à 50) sont agencées au-dessus de la surface supérieure des moyens de support (60 ; 12, 14, 16, 18, 20 ; 40), alors que, dans un second état, les surfaces supérieures des éléments de support (62 ; 13, 15, 17, 19 ; 41 à 50) sont agencées au-dessous de la surface supérieure des moyens de support (60 ; 12, 14, 16, 18, 20 ; 40).

3. Dispositif de désinfection selon l'une quelconque des revendications 1 à 2, comprenant, en outre, des moyens de déplacement configurés de manière à déplacer les moyens de support auxiliaires (62 ; 13, 15, 17, 19 ; 41 à 50) suivant un mouvement continu vers le haut et vers le bas, en particulier, à une fréquence suffisante afin d'amener l'objet dans un état de vibration.

4. Dispositif de désinfection selon l'une quelconque des revendications 1 à 3, dans lequel les éléments de support d'un premier ensemble sont séparés l'un de l'autre et sont mis en œuvre sous forme de colonnes (41 à 50).

5. Dispositif de désinfection selon la revendication 4, dans lequel les colonnes sont agencées suivant une rangée.

6. Dispositif de désinfection selon l'une quelconque des revendications 1 à 3, dans lequel les éléments de support d'un premier ensemble sont séparés l'un de l'autre et sont mis en œuvre sous forme de plaque (13, 15, 17, 19).

7. Dispositif de désinfection selon l'une quelconque des revendications 1 à 3, dans lequel les éléments de support sont reliés mutuellement les uns aux autres afin de former une grille (62).

8. Dispositif de désinfection selon l'une quelconque des revendications précédentes 1 à 7, dans lequel au moins une source de rayonnement UV est située dans une cavité au-dessous des moyens de support (60 ; 12, 14, 16, 18, 20 ; 40), lesquels moyens de support (60 ; 12, 14, 16, 18, 20 ; 40) sont configurés afin d'assurer la transmission du rayonnement UV vers un objet placé sur les moyens de support (60 ; 12, 14, 16, 18, 20 ; 40) et/ou les moyens de support auxiliaires (62 ; 13, 15, 17, 19 ; 41 à 50).

9. Dispositif de désinfection selon la revendication 8, dans lequel les moyens de support (60 ; 12, 14, 16, 18, 20 ; 40) comportent une surface supérieure discontinue et un corps sur lequel ladite surface supérieure discontinue est raccordée, et dans lequel la surface supérieure discontinue est sous la forme d'une grille, d'une série de plaques ou d'une pluralité de colonnes.

10. Dispositif de désinfection selon l'une quelconque des revendications précédentes, comprenant, en outre :
une caméra agencée afin d'enregistrer une image de l'objet,
un moyen d'analyse destiné à analyser l'image de manière à identifier les premières zones de l'objet au niveau desquelles l'objet est supporté par les moyens de support (60 ; 12, 14, 16, 18, 20 ; 40), et à identifier des zones de surface supplémentaires au niveau desquelles l'objet peut être supporté par des éléments de support des moyens de support auxiliaires (62 ; 13, 15, 17, 19 ; 41 à 50) et
un moyen de commande destiné à commander sélectivement des moyens de support auxiliaires (62 ; 13, 15, 17, 19 ; 41 à 50) correspondant aux zones de surface supplémentaires.

11. Dispositif de désinfection selon l'une quelconque des revendications précédentes, comprenant, en outre, un revêtement d'un agent photocatalyseur, tel qu'un matériau semi-conducteur, tel que du TiO2.

12. Dispositif de désinfection selon la revendication 11, dans lequel le revêtement photocatalyseur est formé sur une première face d'un support qui est transparent au rayonnement UV, lequel support comprend, en outre, une seconde face, dans lequel la première face est orientée face à au moins une source de rayonnement UV et dans lequel la seconde face constitue une paroi interne du compartiment de désinfection.

13. Dispositif de désinfection selon l'une quelconque des revendications précédentes, dans lequel le compartiment de désinfection comporte des réflecteurs destinés à réfléchir le rayonnement.

14. Dispositif de désinfection selon l'une quelconque des revendications précédentes, comprenant, en outre, des injecteurs de pulvérisation destinés à pulvériser un liquide sur l'objet et avec celui-ci à éliminer toute matière infectieuse.

15. Utilisation du dispositif de désinfection selon l'une quelconque des revendications précédentes afin d'assurer la désinfection d'un objet.
